# FASCICULE DE BREVET EUROPEEN

(11) **EP 1 702 025 B1**
(45) Date de publication et mention de la délivrance du brevet: **23.02.2011**
(21) Numéro de dépôt: 04817593.9
(22) Date de dépôt: 24.12.2004
(51) Int. Cl.: C10G 2/00, C07C 1/04

(54) **PROCEDE DE TRANSFORMATION D’UN GAZ DE SYNTHESE EN HYDROCARBURES EN PRESENCE DE SIC BETA ET EFFLUENT DE CE PROCEDE**
VERFAHREN ZUR UMWANDLUNG VON SYNTHESEGAS IN KOHLENWASSERSTOFFE IN GEGENWART VON BETA-SIC UND AUSTRAGSSTROM DES VERFAHRENS
METHOD FOR CONVERTING A SYNTHESIS GAS INTO HYDROCARBONS IN THE PRESENCE OF BETA-SIC AND THE EFFLUENT OF SAID METHOD

(30) Priorité: 31.12.2003 FR 0315622
(43) Date de publication de la demande: 20.09.2006
(73) Titulaire: TOTAL RAFFINAGE MARKETING, 92800 Puteaux (FR); Total S.A., 92400 Courbevoie (FR)
(72) Inventeur: SAVIN-PONCET, Sabine, F-64160 Buros (FR); LEDOUX, Marc-Jacques, F-67000 Strasbourg (FR); PHAM-HUU, Cuong, F-67700 Saverne (FR); BOUSQUET, Jacques, F-69540 Irigny (FR); MADANI, Behrang, Bakersfield, CA 93389 (US)
(74) Mandataire: Vieillefosse, Jean-Claude
(86) Numéro de dépôt international: PCT/FR2004/003380
(87) Numéro de publication internationale: WO 2005/073345

(56) Documents cités:
- US-A- 5 585 316
- US-A- 5 648 312

## Description

### DOMAINE TECHNIQUE

La présente invention concerne un procédé de conversion de gaz de synthèse en hydrocarbures C₂⁺ par synthèse Fischer-Tropsch. Elle concerne aussi un procédé de synthèse Fischer-Tropsch produisant un effluant présentant une distribution de la fraction liquide particulière, ainsi que cet effluent.

### ART ANTERIEUR

On connaît de nombreux document décrivant la synthèse Fischer-Tropsch (FT) à l'aide de catalyseurs métalliques supportés sur divers supports catalytiques.

Les documents WO-A-0112323 et WO-A-0154812 (Battelle) citent les carbures de façon générique comme support ou comme couche interfaciale de systèmes catalytiques pour synthèse FT. Il n'y a aucune mention du type de carbure susceptible d'être utilisé.

Les brevets US-P-5648312, US-P-5677257 et US-P-5710093 (Intevep) décrivent un support catalytique particulier adapté à la synthèse FT (susceptible d'être mise en oeuvre dans un lit fixe, un lit ébullisant ou en "slurry"). L'espèce catalytique dans ces documents est un métal du groupe IVB ou VIII, ou un mélange, notamment zirconium et cobalt.

Ce support est obtenu notamment par la formation d'une suspension de silice et de carbure de silicium dans une solution basique, la formation de gouttes puis leur séparation en sphères, puis le passage des sphères dans une solution acide pour conduire à un support catalytique comprenant un mélange sensiblement, homogène de silice et de SiC. Les procédés de synthèse d'hydrocarbures à partir de gaz de synthèse utilisent ce support catalytique, qui est un mélange homogène de particules de silice et de SiC, en une quantité de 10 à 50% du support, le support ayant une surface spécifique d'au moins environ 30 m²/g (de préférence supérieure à 40 m²/g), des diamètres de pores moyens d'au moins environ 150 Å, et une taille de particules d'au moins 0,1 mm. D'après la synthèse, le SiC est nécessairement de forme alpha dans ces documents, le liant de ce SiC étant la silice. La surface spécifique est conférée par le liant silice plutôt que par la forme du SiC; celui-ci est utilisé en fait plus en tant que "puits de chaleur" plutôt que comme support en tant que tel.

La présence de silice et/ou d'alumine en tant que liant peut influencer de façon négative la conductibilité thermique du support. En outre, ce liant comme support présente en outre l'inconvénient de réagir avec l'espèce catalytique, entraînant ainsi une perte de l'activité avec le temps.

### RESUME DE L'INVENTION

L'invention fournit un nouveau procédé de synthèse FT utilisant un nouveau support catalytique comprenant du SiC ß. On obtient ainsi, par rapport aux supports de l'art antérieur, un support particulièrement adapté à la réaction exothermique de la synthèse FT.

Ainsi, l'invention fournit un procédé de transformation de monoxyde de carbone en hydrocarbures C₂⁺ en présence d'hydrogène et d'un catalyseur comprenant un métal et un support contenant du carbure de silicium caractérisé en ce que le support contient plus de 50% en poids de carbure de silicium sous forme bêta.

### BREVE DESCRIPTION DES DESSINS

L'invention est décrite plus en détails en référence aux dessins annexés, dans lesquels:
- la figure 1A donne les résultats de rendements des différentes fractions en fonction du temps sous flux pour un premier mode de réalisation;
- la figure 1B donne la distribution des hydrocarbures à l'intérieur de la fraction liquide pour ce premier mode de réalisation;
- la figure 2A donne les résultats de rendements des différentes fractions en fonction du temps sous flux pour un second mode de réalisation;
- la figure 2B donne la distribution des hydrocarbures à l'intérieur de la fraction liquide pour ce second mode de réalisation;
- les figures 3A et 3B donnent les résultats de rendements des différentes fractions en fonction du temps sous flux pour des troisième et quatrième modes de réalisation;
- la figure 4A donne les résultats de rendements des différentes fractions en fonction du temps sous flux pour un cinquième mode de réalisation;
- la figure 4B donne la distribution des hydrocarbures à l'intérieur de la fraction liquide pour ce cinquième mode de réalisation.

### DESCRIPTION DETAILLEE DE MODES DE REALISATION DE L'INVENTION

Le SiC bêta est préparé par une réaction gaz/solide entre du SiO vapeur et du carbone solide intimement mélangés (sans liquide). Pour plus de détails sur le SiC ß, on pourra se référer aux demandes de brevets et brevets suivants, incorporés par référence à la présente demande: EP-A-0 313480, EP-A-0 440569, US-P-5217930, EP-A-0 511919, EP-A-0 543751 et EP-A-0 543752. Par rapport à la forme alpha, le SiC ß se caractérise notamment par le fait qu'il existe à l'état pur sans liant. Les cristaux sont de type cubique face centrée. En général, la surface spécifique du SiC ß est entre 5 et 40 m²/g et de préférence entre 10 et 25 m²/g.

Le SiC ß peut être préparé sous forme de poudre, de grains, d'extrudés (sans liant), de mousse, de monolithe, etc. La taille du SiC est variable, en fonction du type de procédé mis en oeuvre (lit fixe, ébullisant, "slurry"). On peut ainsi, selon une variante, utiliser une taille comprise entre 0,1 et 20 mm, de préférence entre 1 et 15 mm. Selon une autre variante, on peut utiliser une taille comprise entre 1 et 200 µm, de préférence entre 5 et 150 µm.

Ce SiC ß a des propriétés mécaniques très bonnes. Du fait de sa très bonne conductivité thermique, en général très supérieure à celle des oxydes métalliques, on limite les points chauds à la surface du catalyseur. On améliore ainsi la sélectivité.

Selon un mode de réalisation, le support du catalyseur contient de 50 à 100% en poids de carbure de silicium bêta à l'état particulaire, et de préférence 100% du dit carbure de silicium.

En tant que métal catalytique principal, on peut utiliser de façon classique les métaux du groupe VIII, par exemple notamment le cobalt, le fer ou le ruthénium, le cobalt étant particulièrement préféré. On peut aussi utiliser en même temps un promoteur, de façon classique. Parmi les promoteurs, on peut citer un autre métal du groupe VIII ou encore les métaux choisis dans le groupe consistant en Zr, K, Na, Mn, Sr, Cu, Cr, W, Re, Pt, Ir, Rh, Pd, Ru, Ta, V, Mo et leurs mélanges. Mo est préféré.

La teneur en métal principal, notamment cobalt, est classiquement supérieure à 5%, typiquement entre 10 et 50% du poids final du catalyseur, en particulier entre 20 et 35% en poids. La teneur en promoteur, notamment molybdène, est classiquement entre 0,1 et 15% du poids final du catalyseur, en particulier entre 2 et 10% en poids. Un ratio en poids métal primaire/promoteur est classiquement de 10:1 à 3:1.

Le dépôt du métal catalytique se fait de façon conventionnelle. Par exemple on peut utiliser l'imprégnation du volume poreux par un sel du métal, par exemple du nitrate de cobalt. On peut aussi utiliser la méthode de la goutte évaporée (dite aussi "egg shell"), par goutte à goutte d'une solution de sel métallique à température ambiante sur un support à température élevée conduisant à un dépôt essentiellement en surface, par exemple une solution de nitrate de cobalt sous air sur un support à 200°C.

Le lit catalytique peut être fixe, ébullisant ou en "slurry". On préférera un lit fixe.

La réaction de synthèse de Fischer-Tropsch est en général réalisée dans les conditions opératoires suivantes:
- pression totale: 10 à 100, de préférence 20 à 50 atmosphères;
- température de réaction: 160 à 250, de préférence 180 à 220°C;
- VVH (GHSV) variant de 150 à 5000 h⁻¹, de préférence de 200 à 1000h⁻¹;
- ratio H₂/CO du gaz de synthèse de départ compris entre 1,2 et 2,8, de préférence entre 1,7 et 2,3.

La présente invention concerne également un procédé de synthèse FT qui produit une effluent hydrocarboné qui comprend au moins 70% en mole de C₅ en C₂₅ et dans lequel la distribution est centrée sur des hydrocarbures relativement légers, autour de C₇ à C₁₀. Ce type de distribution n'est pas classique, en particulier pour un procédé mis en oeuvre en lit fixe.

Selon un mode de réalisation, l'effluent hydrocarboné contient plus de 70% en mole d'un mélange comprenant de 50 à 90% en mole d'hydrocarbures de C₆ à C₁₂ et de 10 à 50% d'hydrocarbures de C₁₃ à C₂₄.

Selon un mode de réalisation, l'effluent hydrocarboné contient au plus 10% en mole d'oléfines et d'hydrocarbures ramifiés, et/ou moins de 2% en mole d'alcool en C₁ à C₂₀.

Selon un mode de réalisation, dans l'effluent hydrocarboné la teneur en méthane et en CO₂ est inférieure à 20% en mole.

Dans la demande, les ratios sont molaires sauf mention du contraire.

Les exemples suivants illustrent l'invention sans la limiter.

### Exemple 1.

### Synthèse de Fischer-Tropsch sur un catalyseur à base de cobalt supporté sur du SiC ß.

Dans cet exemple le support à base de SiC ß, sous forme de grains de diamètre compris entre 0,4 et 1 mm, est imprégné par la méthode du volume poreux avec une solution aqueuse contenant le sel de cobalt sous forme nitrate. La masse du sel précurseur est calculée pour avoir une charge finale en cobalt de 30% en poids par rapport au poids de catalyseur après calcination et réduction. Le produit imprégné est séché ensuite sous air à 100°C pendant 2 h puis calciné sous air à 350°C pendant 2 h afin de transformer le sel précurseur en son oxyde correspondant. Le produit après calcination est réduit sous flux d'hydrogène à 400°C pendant 2 h pour obtenir la forme métallique de la phase active.

La réaction de synthèse de Fischer-Tropsch est réalisée dans les conditions suivantes:
- pression totale: 40 atmosphères
- température de réaction: 200°C
- temps de contact réactifs/catalyseur: 12 sec.
- ratio molaire H₂/CO de 2.

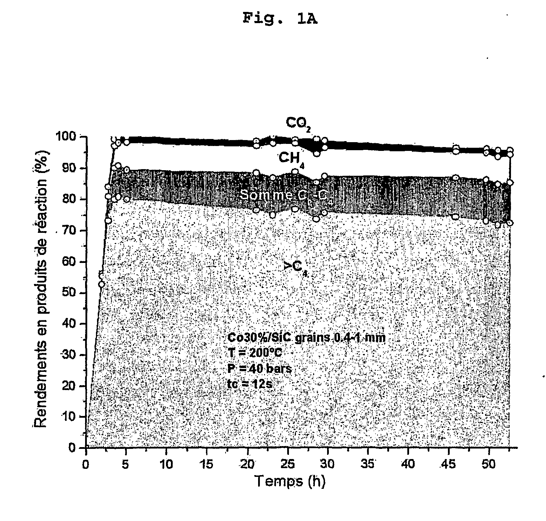

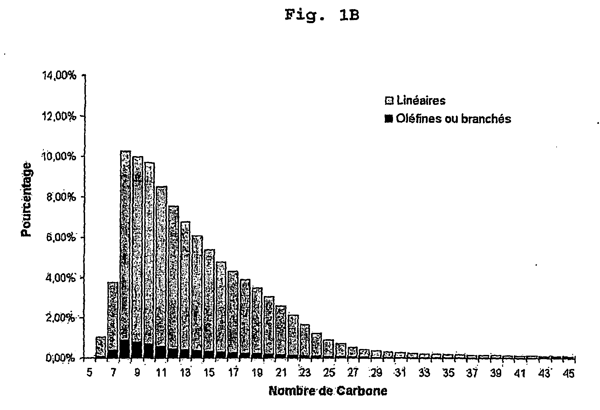

Les résultats obtenus, exprimés en terme de rendements des différentes fractions sont présentés sur la figure 1A en fonction du temps sous flux. Comme on peut le constater le catalyseur à base de Co/SiC ß présente une très bonne activité pour la formation des hydrocarbures liquides à partir du mélange CO/H₂. La distribution des hydrocarbures à l'intérieur de la fraction liquide (C₄⁺) est présentée sur la figure 1B. D'après les résultats obtenus la fraction liquide est essentiellement constituée par des hydrocarbures paraffiniques allant de C₆ à C₂₅, i.e. > 90%, en particulier on note une distribution remarquable d'hydrocarbures légers de la coupe des essences, avec des taux supérieurs à 6% pour les C₇, C₈, C₉, C₁₀, C₁₁, C₁₂ et C₁₃, et des taux supérieurs à 8% pour les C₈, C₉, C₁₀, et C₁₁, tandis que la concentration des hydrocarbures oléfiniques reste faible (moins de 1% en moyenne).

### Exemple 2.

### Influence du molybdène comme promoteur sur l'activité de synthèse de Fischer-Tropsch d'un catalyseur à base dé cobalt supporté sur du SiC ß.

Dans cet exemple on note les conséquences sur les performances du catalyseur Co/SiC ß en présence d'un promoteur qui est du molybdène. Dans cet exemple le support à base de SiC ß, sous forme de grains de diamètre compris entre 0,4 et 1 mm, est imprégné par la méthode de volume poreux avec une solution aqueuse contenant le sel de cobalt sous forme nitrate et une solution aqueuse contenant un ammonium molybdate tétrahydrate. La masse du sel précurseur est calculée pour avoir une charge finale en cobalt de 30% en poids par rapport au poids de catalyseur et une charge de molybdène de 5% en poids par rapport au poids du catalyseur après calcination et réduction. La préparation du catalyseur est la même que dans l'exemple 1.

La réaction de synthèse de Fischer-Tropsch est réalisée dans les mêmes conditions que dans l'exemple 1.

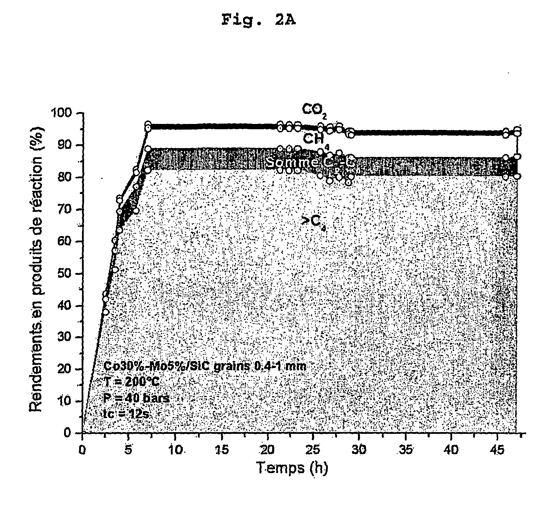

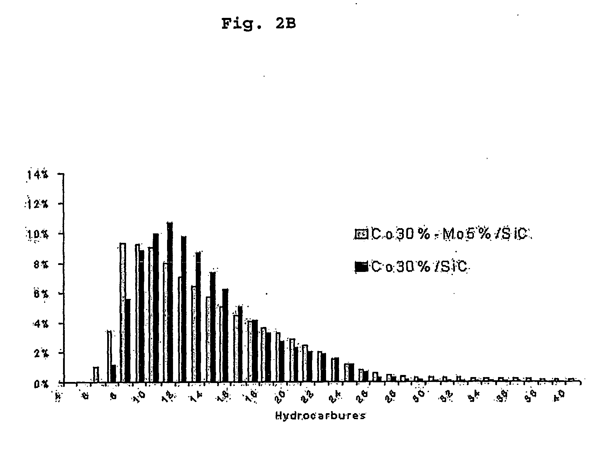

Les résultats obtenus à une température de réaction de 200°C, exprimés en terme de rendement, sont présentés sur la figure 2A en fonction du temps sous flux. Par comparaison avec ceux obtenus à l'exemple 1, on constate que l'addition de molybdène a permis d'augmenter d'une part la stabilité de l'activité et d'autre part d'une manière sensible le rendement en hydrocarbure liquide au détriment des fractions légères telles que CH₄, CO₂ et C₂-C₄. En présence du molybdène la distribution des hydrocarbures dans la fraction liquide est légèrement décalée vers les hydrocarbures de chaînes plus courtes tandis que la distribution des fractions oléfiniques/branchées demeure inchangée. Ceci ressort de la figure 2B, qui représente la distribution des hydrocarbures à l'intérieur de la fraction liquide (C₄⁺).

La concentration des hydrocarbures oléfiniques et branchés reste de même faible (moins de 1% en moyenne).

### Exemple 3.

### Influence de la température de réaction sur l'activité en synthèse de Fischer-Tropsch sur les catalyseurs à base de Co et de Co avec promoteur Mo.

Dans cet exemple le support à base de SiC ß est identique à celui de l'exemple 2.

La réaction de synthèse de Fischer-Tropsch est réalisée dans les conditions suivantes:
- pression totale: 40 atmosphères
- température de réaction: 200°C et 210°C
- temps de contact réactifs/catalyseur: 12 sec.

En présence du molybdène le rendement de la fraction contenant des hydrocarbures liquides est toujours plus élevé comparé à celui obtenu en absence du molybdène sur le catalyseur et ce quelque soit la température de réaction. La présence du molybdène réduit la formation des produits légers tels que CO₂ et CH₄ et favorise la formation des hydrocarbures liquides, et ce quelque soit la température de réaction, i.e. 200°C ou 210°C. Il est à noter également qu'en présence du molybdène l'activité en synthèse Fischer-Tropsch du catalyseur est plus stable qu'en l'absence de molybdène.

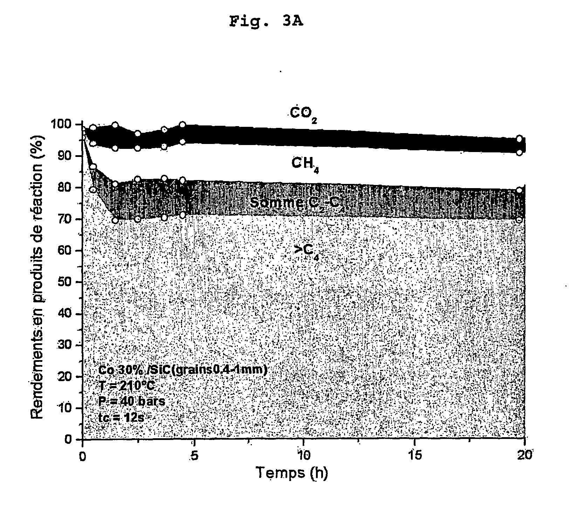

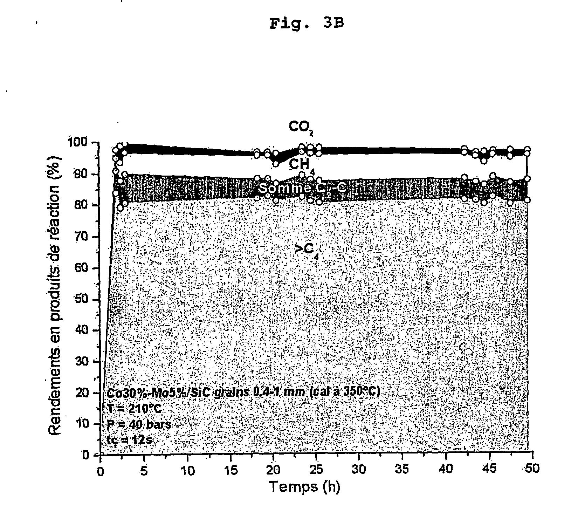

Les différents résultats en terme de rendements des différentes fractions lors de la synthèse de Fischer-Tropsch sont donnés aux figures 1A, 2A, 3A et 3B.

### Exemple 4.

### Synthèse de Fischer-Tropsch sur un catalyseur à base de cobalt supporté sur une monolithe de SiC ß.

Dans cet exemple le support à base de SiC ß, sous forme de monolithe avec une taille moyenne d'ouverture de cellule d'environ 500 µm, est imprégné par la méthode de volume poreux avec une solution aqueuse contenant le sel de cobalt sous forme nitrate. Le procédé d'imprégnation est le même qu'à l'exemple 1.

La réaction de synthèse de Fischer-Tropsch est réalisée dans les conditions suivantes:
- pression totale: 40 atmosphères
- température de réaction: 220°C
- temps de contact réactifs/catalyseur: 12 sec.

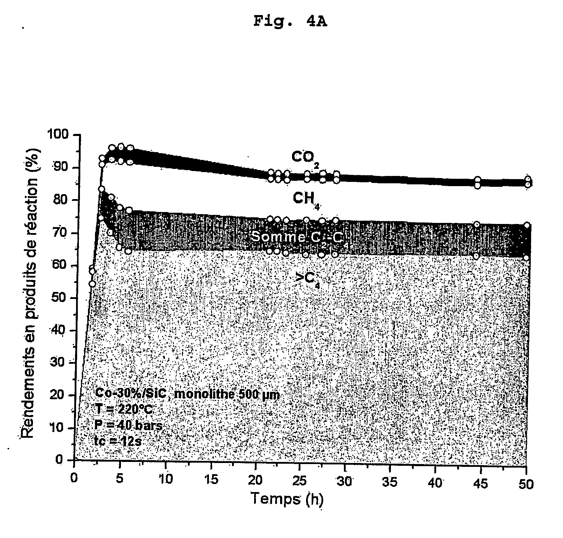

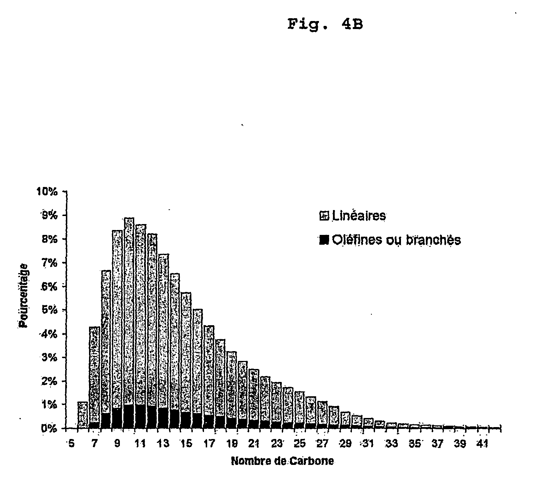

Les résultats obtenus, exprimés en terme de rendements des différentes fractions sont présentés sur la figure 4A en fonction du temps sous flux. La figure 4B donne la distribution des hydrocarbures à l'intérieur de la fraction liquide (C₄⁺). Le rendement en produits liquides reste élevé malgré la température de réaction de 220°C. Ceci est attribué à la forte conductibilité thermique du support à base de SiC ß, évitant les points chauds et améliorant la sélectivité. Les résultats sont de même nature que ceux obtenus à l'exemple 1.

Dans les exemples qui précédent, l'analyse à très haute définition met en évidence des alcools en C₁ à C₁₀, non quantifiables: l'effluent est sensiblement exempt d'alcool.

## Revendications

1. Procédé de transformation de monoxyde de carbone en hydrocarbures C₂ + en présence d'hydrogène et d'un catalyseur comprenant un métal et un support contenant du carbure de silicium **caractérisé en ce que** le support contient plus de 50% en poids de carbure de silicium sous forme bêta.

2. Procédé selon la revendication 1 **caractérisé en ce que** le support du catalyseur contient de 50 à 100% en poids de carbure de silicium bêta à l'état particulaire, et de préférence 100% du dit carbure de silicium.

3. Procédé selon la revendication 1 ou 2, **caractérisé en ce que** le catalyseur contient au moins 5% en poids d'un métal du groupe constitué par le cobalt, le fer ou le ruthénium.

4. Procédé selon l'une des revendications 1 à 3 **caractérisé en ce que** le catalyseur contient en outre un promoteur choisi dans le groupe consistant en Zr, K, Na, Mn, Sr, Cu, Cr, W, Re, Pt, Ir, Rh, Pd, Ru, Ta, V, Mo et leurs mélanges, de préférence Mo.

5. Procédé selon l'une des revendications 1 à 4 **caractérisé en ce que** le SiC bêta est sous forme de poudre, de grains, d'extrudés, de mousse ou de monolithe.

6. Procédé selon l'une des revendications 1 à 5 **caractérisé en ce que** le catalyseur est utilisé en lit fixe, en lit ébullisant ou en slurry.

7. Procédé selon l'une des revendications 1 à 6 **caractérisé en ce qu'**on opère dans les conditions opératoires suivantes:
- pression totale: 10 à 100 atmosphères;
- température de réaction: 160 à 250°C;
- VVH (GHSV) variant de 150 à 5000 h⁻¹;
- ratio H₂/CO du gaz de synthèse de départ compris entre 1,2 et 2,8.

8. Procédé selon l'une des revendications 1 à 7 **caractérisé en ce qu'**on opère dans les conditions opératoires suivantes:
- pression totale: 20 à 50 atmosphères;
- température de réaction: 180 à 220°C;
- VVH (GHSV) variant de 200 à 1000h⁻¹;
- ratio H₂/CO du gaz de synthèse de départ compris entre 1,7 et 2,3.

9. Procédé selon l'une des revendications 1 à 8 **caractérisé en ce que** l'effluent hydrocarboné contient plus de 70% en mole d'un mélange comprenant de 50 à 90% en mole d'hydrocarbures de C₆ à C₁₂ et de 10 à 50% d'hydrocarbures de C₁₃ à C₂₄.

10. Procédé selon l'une des revendications 1 à 9 **caractérisé en ce que** l'effluent hydrocarboné contient au plus 10% en mole d'oléfines et d'hydrocarbures ramifiés, et/ou moins de 2% en mole d'alcool en C₁ à C₂₀.

11. Procédé selon l'une des revendications 1 à 9 **caractérisé en ce que** dans l'effluent hydrocarboné la teneur en méthane et en CO₂ est inférieur à 20% en mole.

## Claims

1. A method for transforming carbon monoxide into C₂⁺ hydrocarbons in the presence of hydrogen and of a catalyst comprising a metal and a support containing silicon carbide **characterized in that** the support contains more than 50% by weight of silicon carbide in beta form.

2. The method according to claim 1, **characterized in that** the support of the catalyst contains from 50 to 100% by weight of beta silicon carbide in a particulate state, and preferably 100% of said silicon carbide.

3. The device according to claim 1 or 2, **characterized in that** the catalyst contains at least 5% by weight of a metal from the group consisting of cobalt, iron or ruthenium.

4. The method according to claims 1 to 3, **characterized in that** the catalyst further contains a promoter selected from the group consisting of Zr, K, Na, Mn, Sr, Cu, Cr, W, Re, Pt, Ir, Rh, Pd, Ru, Ta, V, Mo and their mixtures, preferably Mo.

5. The method according to any of claims 1 to 4, **characterized in that** the beta SiC is in the form of powder, grains, extrudates, foam or monolith.

6. The method according to any of claims 1 to 5, **characterized in that** the catalyst is used in a fixed bed, in a boiling or slurry bed.

7. The method according to any of claims 1 to 6, **characterized in that** it is performed under the following operating conditions:
- total pressure: 10 to 100 atmospheres;
- reaction temperature: 160 to 250°C;
- VVH (GHSV) varying from 150 to 5,000 h⁻¹;
- H₂/CO ratio of the starting synthesis gas comprised between 1.2 and 2.8.

8. The method according to any of claims 1 to 7, **characterized in that** it is performed under the following operating conditions:
- total pressure: 20 to 50 atmospheres;
- reaction temperature: 180 to 220°C;
- VVH (GHSV) varying from 200 to 1,000 h⁻¹;
- H₂/CO ratio of the starting synthesis gas comprised between 1.7 and 2.3.

9. The method according to any of claims 1 to 8, **characterized in that** the hydrocarbon effluent contains more than 70% by moles of a mixture comprising 50 to 90% by moles of C₆-C₁₂ hydrocarbons and from 10 to 50% of C₁₃-C₂₄ hydrocarbons.

10. The method according to any of claims 1 to 9, **characterized in that** the hydrocarbon effluent contains at most 10% by moles of branched hydrocarbons and olefins and/or at least 2% by moles of a C₁-C₂₀ alcohol.

11. The method according to any of claims 1 to 9, **characterized in that** the methane and CO₂ content in the hydrocarbon effluent is less than 20% by moles.

## Patentansprüche

1. Verfahren zur Umwandlung von Kohlenmonoxid in C₂⁺-Kohlenwasserstoffe in Gegenwart von Wasserstoff und eines Katalysators umfassend ein Metall und einen Träger enthaltend Siliciumcarbid, **dadurch gekennzeichnet, dass** der Träger mehr als 50 Gewichts% an Siliciumcarbid in der Beta-Form enthält.

2. Verfahren nach Anspruch 1, **dadurch gekennzeichnet, dass** der Träger des Katalysators 50 bis 100 Gewichts% an beta-Siliciumcarbid in partikelartigem Zustand, und vorzugsweise 100 % dieses Siliciumcarbids, enthält.

3. Verfahren nach Anspruch 1 oder 2, **dadurch gekennzeichnet, dass** der Katalysator mindestens 5 Gewichts% eines Metalls der Gruppe bestehend aus Kobalt, Eisen oder Ruthenium enthält.

4. Verfahren nach einem der Ansprüche 1 bis 3, **dadurch gekennzeichnet, dass** der Katalysator weiterhin einen Hilfsstoff enthält, der aus der Gruppe bestehend aus Zr, K, Na, Mn, Sr, Cu, Cr, W, Re, Pt, Ir, Rh, Pd, Ru, Ta, V, Mo und deren Mischungen gewählt ist, vorzugsweise Mo.

5. Verfahren nach einem der Ansprüche 1 bis 4, **dadurch gekennzeichnet, dass** das beta-SiC in Form von Pulver, Körnchen, Extrudatstücken, Schaum oder als Monolith vorliegt.

6. Verfahren nach einem der Ansprüche 1 bis 5, **dadurch gekennzeichnet, dass** der Katalysator als Festbett, Fließbett oder als Aufschlämmung verwendet wird.

7. Verfahren nach einem der Ansprüche 1 bis 6, **dadurch gekennzeichnet, dass** die folgenden Betriebsbedingungen zur Anwendung kommen:
- Gesamtdruck: 10 bis 100 Atmosphären;
- Reaktionstemperatur: 160 bis 250 °C;
- VVH (GHSV) im Bereich von 150 bis 5.000 h⁻¹;
- H₂/CO-Verhältnis des Ausgangs-Synthesegases im Bereich von 1,2 bis 2,8.

8. Verfahren nach einem der Ansprüche 1 bis 7, **dadurch gekennzeichnet, dass** die folgenden Betriebsbedingungen zur Anwendung kommen:
- Gesamtdruck: 20 bis 50 Atmosphären;
- Reaktionstemperatur: 180 °C bis 220 °C;
- VVH (GHSV) im Bereich von 200 bis 1.000h h⁻¹;
- H₂/CO-Verhältnis des Ausgangs-Synthesegases im Bereich von 1,7 bis 2,3.

9. Verfahren nach einem der Ansprüche 1 à 8, **dadurch gekennzeichnet, dass** der kohlenwasserstoffhaltige Stoffstrom mehr als 70 Mol% einer Mischung enthält, die 50 bis 90 Mol% an C₆-C₁₂-Kohlenwasserstoffen und 10 bis 50 % an C₁₃- bis C₂₄-Kohlenwasserstoffen umfasst.

10. Verfahren nach einem der Ansprüche 1 bis 9, **dadurch gekennzeichnet, dass** der kohlenwasserstoffhaltige Stoffstrom höchstens 10 Mol% an verzweigten Olefinen und Kohlenwasserstoffen und/oder weniger als 2 Mol% an C₁-C₂₀-Alkohol enthält.

11. Verfahren nach einem der Ansprüche 1 bis 9, **dadurch gekennzeichnet, dass** im kohlenwasserstoffhaltigen Stoffstrom der Gehalt an Methan und an CO₂ geringer als 20 Mol% ist.
